# EUROPEAN PATENT APPLICATION

(11) **EP 4 714 441 A1**
(43) Date of publication of application: **25.03.2026**
(21) Application number: 24849166.4
(22) Date of filing: 30.07.2024
(51) Int. Cl.: A61K 31/385, A23L 33/10, A61P 1/16

(54) **COMPOSITION FOR PREVENTING OR ALLEVIATING HEPATIC DYSFUNCTION**

(30) Priority: 01.08.2023 JP 2023125503
(71) Applicant: KIRIN HOLDINGS KABUSHIKI KAISHA, Nakano-ku, Tokyo 164-0001 (JP); Kyowa Hakko Bio Co., Ltd., Tokyo 100-8185 (JP)
(72) Inventor: SAKO Naoki, Tokyo 164-0001 (JP); MORITA Masahiko, Tokyo 164-0001 (JP); OOSHIMA Etsuo, Tokyo 164-0001 (JP)
(74) Representative: Vossius & Partner Patentanwälte Rechtsanwälte mbB
(86) International application number: PCT/JP2024/027139
(87) International publication number: WO 2025/028521

(57) **Abstract**

Disclosed is a composition for preventing or alleviating hepatic dysfunction, the composition containing a trisulfide compound represented by formula (1) or (6), a pharmaceutically acceptable salt thereof, or a cyclodextrin inclusion of the same.

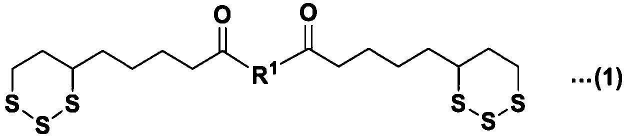

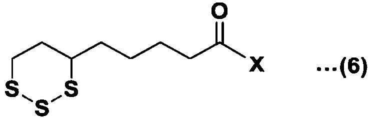

## Description

### Technical Field

The present invention relates to a composition for preventing or improving hepatic dysfunction.

### Background Art

The liver is a reddish-brown organ that is the largest organ of all organs in terms of volume and weight, weighing 1,000 g to 1,500 g in adult males and 900 g to 1,300 g in adult females. The liver contributes to about 40% of the basal metabolism among all organs and plays a wide variety of important roles, such as maintaining respiration, body temperature maintenance, metabolism of nutrients such as carbohydrates, lipids, proteins, and vitamins, and detoxification of harmful xenobiotics or nitrogen metabolites produced by life activities.

A decline in hepatic function can be caused by hepatic dysfunction and various hepatic diseases associated therewith. It is known that as these conditions progress, subjective symptoms such as fatigue, malaise, nausea, loss of appetite, and fever appear, and these conditions can also cause complications associated with the diseases. Examples of hepatic diseases include hepatitis B, hepatitis C, alcoholic liver disease, or non-alcoholic fatty liver disease. These hepatic diseases, along with the increase in lifestyle-related diseases based on obesity, diabetes, dyslipidemia, and the like, have become one of the most common lifestyle-related diseases in modem people (Non Patent Literature 1).

Since hepatic dysfunction, if left untreated, carries a risk of developing into serious diseases such as liver cirrhosis and liver cancer, it is important to protect the liver at an early stage and to appropriately and reliably prevent or improve hepatic dysfunction.

One of the substances known to have a hepatoprotective effect is reduced glutathione (GSH) (Non Patent Literature 2 and 3), and there are also reports that alpha-lipoic acid (LA), which has been shown to produce glutathione in vivo, has a protective effect on hepatocytes (Non Patent Literature 4 and 5).

Furthermore, trisulfide compounds such as glutathione trisulfide are converted in vivo into reactive sulfur species such as glutathione persulfide. It has been reported that reactive sulfur species have a potent antioxidant effect and may have physiological functions such as anti-aging (for example, Non Patent Literature 6 and 7). As a method for producing a trisulfide compound, the method described in Patent Literature 1, for example, is known.

Alpha-lipoic acid trisulfide (LASSS), a type of trisulfide compound, is known to suppress the nitration of tyrosine residues, which leads to the loss of the physiological activity of Hepatocyte Growth Factor (hereinafter also referred to as "HGF"), a growth factor involved in cell proliferation and the like (Patent Literature 2). On the other hand, the effects on hepatic function of administering alpha-lipoic acid trisulfide (LASSS), alpha-lipoic acid trisulfide choline ester (LASSS-CE), or the like have not been known at all.

### Citation List

### Patent Literature

Patent Literature 1: WO 2018/117186
Patent Literature 2: WO 2023/038088

### Non Patent Literature

Non Patent Literature 1: Okanoue, et al., "Liver function tests and liver injury - focusing on AST/ALT and PLT count", Health Evaluation and Promotion, 42(2), 307-312, (2015)
Non Patent Literature 2: Sugimura, et al., "Effect of Orally Administered Reduced- and Oxidized-Glutathione against Acetaminophen-Induced Liver Injury in Rats", Journal of Nutritional Science and Vitaminology, 44, 613-624, (1998)
Non Patent Literature 3: Honda, et al., "Efficacy of glutathione for the treatment of nonalcoholic fatty liver disease: an open-label, single-arm, multicenter, pilot study", BMC Gastroenterology, 17:96, (2017)
Non Patent Literature 4: Kuo, et al., "Alpha-lipoic acid induces adipose triglyceride lipase expression and decreases intracellular lipid accumulation in HepG2 cells", European Journal of Pharmacology, 692:1-3, 10-18, (2012)
Non Patent Literature 5: Shi, et al., "α-Lipoic acid protects against the cytotoxicity and oxidative stress induced by cadmium in HepG2 cells through regeneration of glutathione by glutathione reductase via Nrf2/ARE signaling pathway", Environmental Toxicology and Pharmacology, 45, 274-281, (2016)
Non Patent Literature 6: Ida, et al., "Reactive cysteine persulfides and S-polythiolation regulate oxidative stress and redox signaling", Proceedings of the National Academy of Sciences of the United States of America, 111(21), 7606-7611, (2014)
Non Patent Literature 7: Kunikata et al., "Metabolomic profiling of reactive persulfides and polysulfides in the aqueous and vitreous humors", Scientific Reports, 7, 41984 (2017)

### Summary of Invention

### Technical Problem

An object of the present invention is to provide a composition for preventing or improving hepatic dysfunction.

### Solution to Problem

The present inventors have found that alpha-lipoic acid trisulfide and derivatives thereof improve the cell viability of a human liver cancer-derived cell line (HepG2 cells) that has suffered cell damage due to hydrogen peroxide, and have completed the present invention.
The present invention relates to the following [1] to [14].
[1] A composition for preventing or improving hepatic dysfunction, comprising a trisulfide compound,
   wherein the trisulfide compound is at least one selected from the group consisting of:
   a compound represented by Formula (1) (hereinafter also referred to as compound (1)): 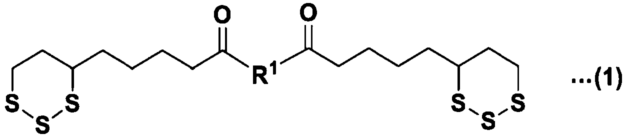
   wherein R¹ is a group represented by Formula (2): 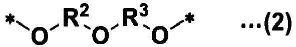
   wherein R² and R³ are each independently a C₁₋₄ alkylene group, and * is a bond;
   a group represented by Formula (3): 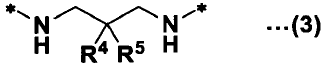
   wherein R⁴ and R⁵ are each independently a hydrogen atom or a C₁₋₄ alkyl group optionally having a substituent, and R⁴ and R⁵ may form a ring together with the atom bonded thereto, and * is a bond;
   a group represented by Formula (4): 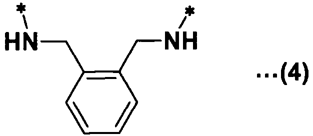
   wherein * is a bond, or
   a group represented by Formula (5): 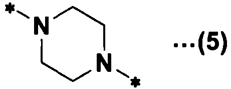
   wherein * is a bond,
   a pharmaceutically acceptable salt thereof, and a cyclodextrin clathrate thereof; and
   a compound represented by Formula (6) (hereinafter also referred to as compound (6)): 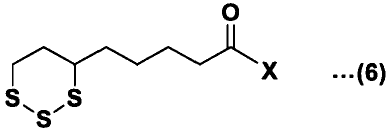
   wherein X is -OR⁶ or -NR⁷R⁸,
   wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
   wherein R⁹, R¹⁰, R¹¹, R¹², R¹³, R¹⁴, and R¹⁵ are each independently a hydrogen atom or a C₁₋₃ alkyl group, and n is an integer of 2 to 5,
   a pharmaceutically acceptable salt thereof, and a cyclodextrin clathrate thereof.
[2] The composition according to [1], wherein the hepatic dysfunction is a condition in which an abnormality is observed in hepatic function without being accompanied by a hepatic disease, or a condition in which an abnormality is observed in hepatic function accompanied by a hepatic disease.
[3] The composition according to [2], wherein the hepatic disease is at least one selected from the group consisting of hepatitis B, hepatitis C, alcoholic liver disease, non-alcoholic fatty liver disease, and chronic hepatitis.
[4] The composition according to [1] or [2], wherein the hepatic dysfunction is due to reactive oxygen species-induced damage.
[5] The composition according to any one of [1] to [4], which is a food composition.
[6] The composition according to any one of [1] to [4], which is a pharmaceutical composition.
[7] The composition according to any one of [1] to [6], wherein the trisulfide compound is alpha-lipoic acid trisulfide or a pharmaceutically acceptable salt thereof, or alpha-lipoic acid trisulfide choline ester or a pharmaceutically acceptable salt thereof.
[8] A method for preventing or improving hepatic dysfunction, comprising administering a trisulfide compound to a subject in need thereof,
   wherein the trisulfide compound is at least one selected from the group consisting of:
   compound (1), a pharmaceutically acceptable salt thereof, and a cyclodextrin clathrate thereof; and
   compound (6), a pharmaceutically acceptable salt thereof, and a cyclodextrin clathrate thereof.
[9] A trisulfide compound for use in preventing or improving hepatic dysfunction,
   wherein the trisulfide compound is at least one selected from the group consisting of:
   compound (1), a pharmaceutically acceptable salt thereof, and a cyclodextrin clathrate thereof; and
   compound (6), a pharmaceutically acceptable salt thereof, and a cyclodextrin clathrate thereof.
   [9a] A non-therapeutic use of a trisulfide compound in preventing or improving hepatic dysfunction,
   wherein the trisulfide compound is at least one selected from the group consisting of:
   compound (1), a pharmaceutically acceptable salt thereof, and a cyclodextrin clathrate thereof; and
   compound (6), a pharmaceutically acceptable salt thereof, and a cyclodextrin clathrate thereof.
[10] Use of a trisulfide compound for the manufacture of a composition for preventing or improving hepatic dysfunction,
   wherein the trisulfide compound is at least one selected from the group consisting of:
   compound (1), a pharmaceutically acceptable salt thereof, and a cyclodextrin clathrate thereof; and
   compound (6), a pharmaceutically acceptable salt thereof, and a cyclodextrin clathrate thereof.
[11] The method, compound for use, or use according to any one of [8] to [10], wherein the hepatic dysfunction is a condition in which an abnormality is observed in hepatic function without being accompanied by a hepatic disease, or a condition in which an abnormality is observed in hepatic function accompanied by a hepatic disease.
[12] The method, compound for use, or use according to [11], wherein the hepatic disease is at least one selected from the group consisting of hepatitis B, hepatitis C, alcoholic liver disease, non-alcoholic fatty liver disease, and chronic hepatitis.
[13] The method, compound for use, or use according to any one of [8] to [11], wherein the hepatic dysfunction is due to reactive oxygen species-induced damage.
[14] The method, compound for use, or use according to any one of [8] to [13], wherein the trisulfide compound is alpha-lipoic acid trisulfide or a pharmaceutically acceptable salt thereof, or alpha-lipoic acid trisulfide choline ester or a pharmaceutically acceptable salt thereof.

### Advantageous Effects of Invention

According to the present invention, a composition for preventing or improving hepatic dysfunction can be provided. The composition for prevention or improvement of the present invention, by comprising at least one trisulfide compound selected from the group consisting of compound (1), a pharmaceutically acceptable salt thereof, and a cyclodextrin clathrate thereof, and compound (6), a pharmaceutically acceptable salt thereof, and a cyclodextrin clathrate thereof, can protect the liver in vivo via a high anti-inflammatory effect or antioxidant effect, and prevent or improve hepatic dysfunction. Furthermore, when the composition of the present invention is a health functional food or a food composition consumed with a focus on the specific functions of the present invention, it can be taken regularly.

### Brief Description of Drawings

Fig. 1 is a diagram showing the effect of adding LASSS or LASSS-CE on HepG2 cells in which hepatic dysfunction was induced by the addition of hydrogen peroxide solution. The left graph shows the cell viability when LASSS was added, and the right graph shows the cell viability when LASSS-CE was added.
Fig. 2 is a diagram showing the effect of adding LASSS or LASSS-CE on HepG2 cells to which hydrogen peroxide solution was not added. The left graph shows the cell viability when LASSS was added, and the right graph shows the cell viability when LASSS-CE was added.

### Description of Embodiments

Hereinafter, the content of the present invention will be described in detail, but the present invention is not limited to the following embodiments.

The trisulfide compound contained in the composition for preventing or improving hepatic dysfunction of the present invention is at least one selected from the group consisting of compound (1), a pharmaceutically acceptable salt thereof, and a cyclodextrin clathrate thereof, and compound (6), a pharmaceutically acceptable salt thereof, and a cyclodextrin clathrate thereof.

Compound (1): 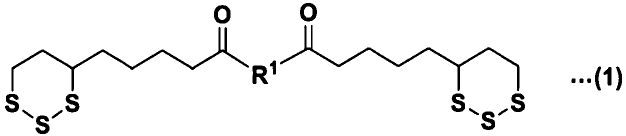 wherein R¹ is a group represented by Formula (2): 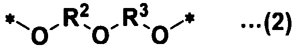
wherein R² and R³ are each independently a C₁₋₄ alkylene group, and * is a bond;
a group represented by Formula (3): 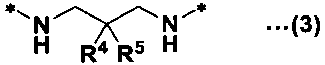
wherein R⁴ and R⁵ are each independently a hydrogen atom or a C₁₋₄ alkyl group optionally having a substituent, and R⁴ and R⁵ may form a ring together with the atom bonded thereto, and * is a bond;
a group represented by Formula (4): 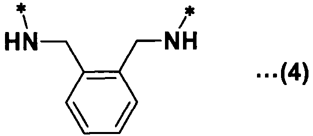
wherein * is a bond, or
a group represented by Formula (5): 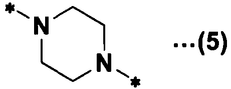
wherein * is a bond.

Compound (6): 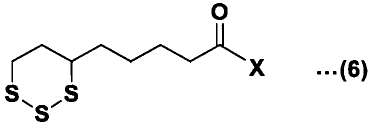
wherein X is -OR⁶ or -NR⁷R⁸,
wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
wherein R⁹, R¹⁰, R¹¹, R¹², R¹³, R¹⁴, and R¹⁵ are each independently a hydrogen atom or a C₁₋₃ alkyl group, and n is an integer of 2 to 5.

Compound (1) can be described as a compound in which two molecules of alpha-lipoic acid trisulfide are bonded via a linker.

In compound (1), R² and R³ are each independently a C₁₋₄ alkylene group. Examples of the C₁₋₄ alkylene group include methylene, ethylene, n-propylene, isopropylene, cyclopropylene, n-butylene, isobutylene, sec-butylene, tert-butylene, cyclobutylene, and the like.

R⁴ and R⁵ are each independently a hydrogen atom or a C₁₋₄ alkyl group optionally having a substituent, and R⁴ and R⁵ may form a ring together with the atom to which they are bonded. Examples of substituents include a hydroxy group; C₁₋₄ alkoxy groups such as methoxy, ethoxy, propoxy, and butoxy. Examples of C₁₋₄ alkyl groups include methyl, ethyl, n-propyl, isopropyl, cyclopropyl, n-butyl, isobutyl, sec-butyl, tert-butyl, cyclobutyl and the like. When R⁴ and R⁵ form a ring together with the atom to which they are bonded, the formed ring may be, for example, an oxetane ring.

Compound (1) can be produced by:
(A) a step of condensing a compound represented by Formula (1a) (lipoic acid trisulfide) with a molecule for forming a linker (a compound represented by Formula (2a), a compound represented by Formula (3a), o-xylene diamine, m-xylene diamine, p-xylene diamine, piperazine, etc.) (Step 1), or
(B) a step of condensing a compound represented by Formula (1b) (lipoic acid) with a molecule for forming a linker as described above (Step 1'), a step of oxidizing the obtained disulfide compound with an oxidizing agent to obtain a sulfoxide compound (Step 2), and a step of allowing the obtained sulfoxide compound to react with a sulfur source to obtain a trisulfide compound (Step 3).

Examples of solvents used in Step 1 and Step 1' include methylene chloride, chloroform, and tetrahydrofuran, and methylene chloride is preferred. The amount of solvent may be 1 mL to 200 mL, preferably 3 mL to 35 mL, per 1 g of compound (1a) or compound (1b). Examples of condensing agents used in Step 1 and Step 1' include 1-(3-dimethylaminopropyl)-3-ethylcarbodiimide (EDC) and a salt thereof, N,N'-dicyclohexylcarbodiimide (DCC), diisopropylcarbodiimide (DIC), 4-dimethylaminopyridine (DMAP), 1,1'-carbonyldiimidazoledi(1H-imidazole-1-yl)methanone (CDI) and the like. When using these compounds as condensing agents, additives such as N-hydroxysuccinimide (NHS) and 1-hydroxybenzotriazole (HOBt) may be used. The amount of condensing agent used in Step 1 and Step 1' may be 0.8 equivalents to 2.0 equivalents, preferably 1.0 equivalent to 1.5 equivalents, per 1 equivalent of compound (1a) or compound (1b). The reaction temperature in Step 1 and Step 1' may be -10°C to 40°C, preferably 15°C to 25°C. The reaction time in Step 1 and Step 1' may be 1 hour to 3 days, preferably 1 hour to 24 hours.

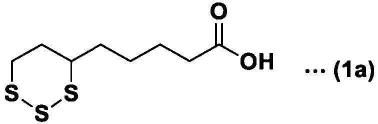

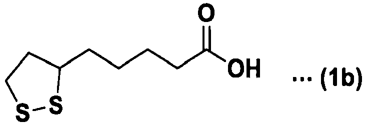

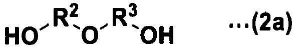

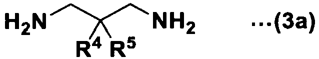

To link two molecules of compound (1a) or compound (1b) via a linker, the amount of the molecule for forming the linker used in Step 1 and Step 1' may be 0.45 to 1.00 equivalents relative to the amount of lipoic acid trisulfide. However, other equivalents may be used as long as the desired compound can be obtained.

The compound represented by Formula (1a) (lipoic acid trisulfide) can be produced by the method described in WO 2022/045212. The procedures and conditions for Steps 2 and 3 in production method (B) can be, for example, in accordance with the procedures and conditions for Steps 1 and 2 in WO 2022/045212.

R² and R³ in Formula (2a) are the same as R² and R³ in Formula (2), respectively. Examples of compounds represented by Formula (2a) include C₄₋₆ dialkylene glycols. Examples of compounds represented by Formula (2a) include diethylene glycol, linear or branched dipropylene glycol, and the like.

R⁴ and R⁵ in Formula (3a) are the same as R⁴ and R⁵ in Formula (3), respectively. Examples of compounds represented by Formula (3a) include 2,2-dimethyl-1,3-propanediamine, 2-oxetane-1,3-propanediamine, 2-methoxy-1,3-propanediamine, and the like.

Examples of compound (1) include compounds represented by the following Formulae (11) to (16) (compounds (11) to (16)) or the like.

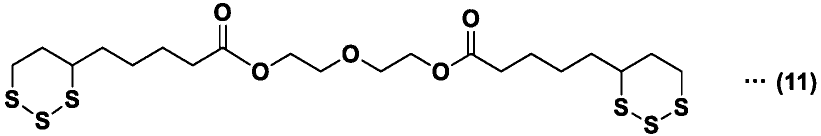

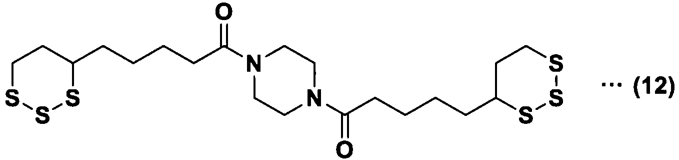

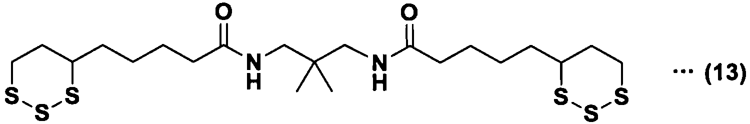

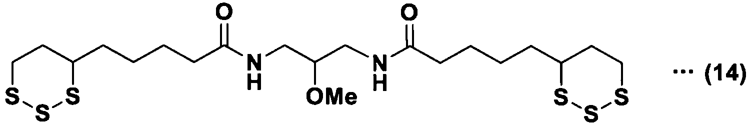

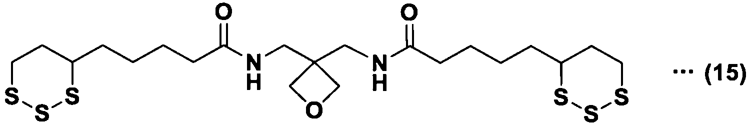

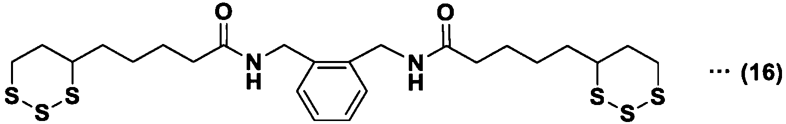

The molecular weights (M.W.) and ClogP values of compounds (11) to (16) are shown in Table 1.

**[Table 1]**

| | Compound (11) | Compound (12) | Compound (13) | Compound (14) | Compound (15) | Compound (16) |
|---|---|---|---|---|---|---|
| M.W. | 546.8 | 526.9 | 542.9 | 544.9 | 556.9 | 576.9 |
| Clog P | 6.512 | 4.792 | 5.585 | 4.568 | 3.381 | 5.732 |

The molecular weight of compound (1) or a salt thereof may be 800 or less, 750 or less, or 700 or less, and is preferably 600 or less. The molecular weight of compound (1) or a salt thereof may be 400 or more, 450 or more, or 500 or more. When the molecular weight of compound (1) or a salt thereof is within these ranges, the *in vivo* absorption rate is considered to be further improved.

The ClogP of compound (1) or a salt thereof may be 1 or more, 2 or more, 3 or more, or 4 or more. The ClogP of compound (1) or a salt thereof may be 8 or less, 7 or less, or 6 or less. When the ClogP of compound (1) or a salt thereof is within these ranges, the balance of water solubility and lipophilicity of compound (1) or a salt thereof is considered to be more excellent. ClogP is a computer-calculated partition coefficient and can be determined in accordance with the principles described in "CLOGP Reference Manual Daylight Version 4.9 (Release date: August 1, 2011, https://www.daylight.com/dayhtml/doc/clogp/)".

The compounds described in this specification may have optical isomers (R-form, S-form, R,R-form, R,S-form, S,R-form, and S,S-form). The compounds described herein may be replaced with their optical isomers or mixtures containing the optical isomers at any ratio (for example, racemates). For example, compound (1) may be an optical isomer represented by Formula (1-1), an optical isomer represented by Formula (1-2), an optical isomer represented by Formula (1-3), or an optical isomer represented by Formula (1-4), or may be replaced with a mixture containing these optical isomers at any ratio (for example, racemate).

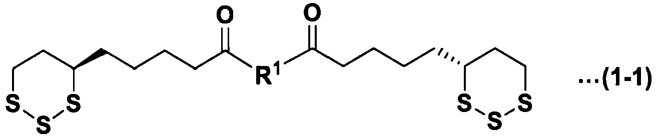

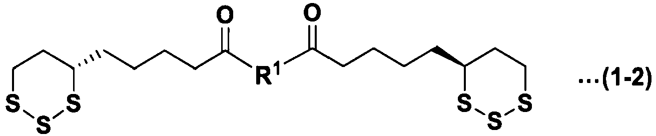

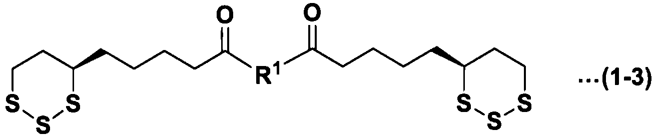

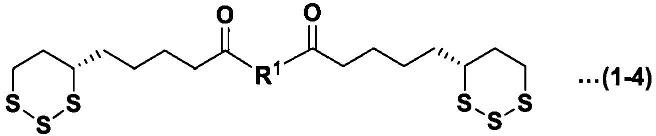

Compound (6) may be, in one embodiment, a compound represented by Formula (7): 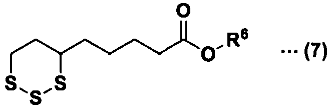 wherein R⁶ is a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5. For example, R⁶ may be a hydrogen atom, methyl, ethyl, propyl, butyl, pentyl, or hexyl. For example, R⁶ is a hydrogen atom, in which case the compound represented by Formula (7) is alpha-lipoic acid trisulfide represented by Formula (8). For example, R⁶ is -(CH₂)₂N⁺(CH₃)₃, in which case the compound represented by Formula (7) is alpha-lipoic acid trisulfide choline ester (LASSS-CE) represented by Formula (8'). Compound (6) is, preferably, a compound selected from the group consisting of alpha-lipoic acid trisulfide (LASSS) and alpha-lipoic acid trisulfide choline ester (LASSS-CE).

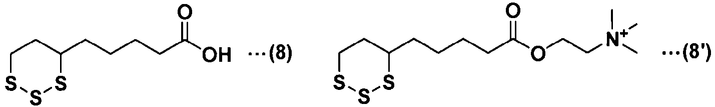

Compound (6) may be, in another embodiment, a compound represented by Formula (9): 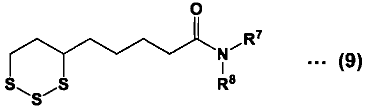 wherein R⁷ and R⁸ are each independently a hydrogen atom; a C_{1- 6} alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5. R⁷ and R⁸ may be a hydrogen atom or an alkyl group such as methyl, ethyl, propyl, butyl, pentyl, or hexyl. These alkyl groups may have one or both of an amino group and a carboxy group as substituents. R⁷ and R⁸ may be, for example, a group represented by Formula (10), wherein * is a bond. Specific examples of the compound represented by Formula (9) include a compound in which both R⁷ and R⁸ are hydrogen atoms, and a compound in which R⁷ is a hydrogen atom and R⁸ is a group represented by Formula (10).

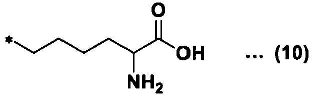

The compound represented by Formula (9) can be produced by a step (Step 61) of oxidizing a compound represented by Formula (9a) with an oxidizing agent to obtain a sulfoxide compound, and a step (Step 62) of reacting the obtained sulfoxide compound with a sulfur source. 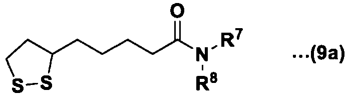 wherein R⁷ and R⁸ are each independently a hydrogen atom; a C_{1- 6} alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5.

In the above production method, Steps 61 and 62 may be carried out in one pot without isolating the sulfoxide compound.

The solvent used in Step 61 is not particularly limited as long as it dissolves the compound represented by Formula (9a) and the oxidizing agent and does not inhibit the oxidation reaction. Examples of such solvents include water, aqueous sulfuric acid solution, aqueous ethanol solution, and aqueous acetonitrile solution, and water is preferred. The amount of solvent used in Step 61 may be 1 mL to 500 mL, preferably 10 mL to 20 mL, per 1 g of the compound represented by Formula (9a).

Examples of oxidizing agents used in Step 61 include potassium peroxymonosulfate (sold under trade names such as Oxone^{®}), peracetic acid, hydrogen peroxide, and sodium periodate. Hydrogen peroxide may be used with a catalytic amount of methyltrioxorhenium. From the viewpoint of safety and cost, potassium peroxymonosulfate is a preferred oxidizing agent. The amount of oxidizing agent used may be 0.8 equivalents to 2.0 equivalents, preferably 1.0 equivalent to 1.3 equivalents, per 1 equivalent of the compound represented by Formula (9a).

The reaction temperature of Step 61 may be -20°C to 30°C, preferably -5°C to 5°C.

The reaction time of Step 61 may be 5 minutes to 24 hours, preferably 0.5 hours to 2 hours.

The solvent used in Step 62 is not particularly limited as long as it dissolves the sulfoxide compound and the sulfur source and does not inhibit the subsequent reaction. Examples of such solvents include water, aqueous sulfuric acid solution, aqueous ethanol solution, and aqueous acetonitrile solution, and water is preferred. The amount of solvent used in Step 62 may be 1 mL to 500 mL, preferably 10 mL to 20 mL, per 1 g of the sulfoxide compound.

Examples of sulfur sources used in Step 62 include sodium sulfide, potassium sulfide, sodium hydrogen sulfide, potassium hydrogen sulfide, and hydrogen sulfide. The amount of sulfur source used may be 0.5 equivalents to 4.0 equivalents, preferably 0.9 equivalents to 1.2 equivalents, per 1 equivalent of the sulfoxide compound.

The reaction temperature of Step 62 may be -20°C to 30°C, preferably -5°C to 25°C.

The reaction time of Step 62 may be 10 minutes to 2 days, preferably 0.5 hours to 2 hours.

When Steps 61 and 62 are carried out in one pot, examples of reaction solvents include water, aqueous sulfuric acid solution, aqueous ethanol solution, and aqueous acetonitrile solution, and water is preferred. The amount of solvent may be 1 mL to 500 mL, preferably 10 mL to 20 mL, per 1 g of the compound represented by Formula (9a). Examples of oxidizing agents used include potassium peroxymonosulfate, peracetic acid, hydrogen peroxide (which may be used with a catalytic amount of methyltrioxorhenium), and sodium periodate, and potassium peroxymonosulfate is preferred. The amount of oxidizing agent used may be 0.8 equivalents to 2.0 equivalents, preferably 1.0 equivalent to 1.3 equivalents, per 1 equivalent of the compound represented by Formula (9a). Examples of sulfur sources used include sodium sulfide, potassium sulfide, sodium hydrogen sulfide, potassium hydrogen sulfide, and hydrogen sulfide. The amount of sulfur source used may be 0.5 equivalents to 4.0 equivalents, preferably 0.9 equivalents to 1.2 equivalents, per 1 equivalent of the compound represented by Formula (9a). The reaction temperature may be -20°C to 30°C, preferably -5°C to 25°C. The reaction time may be 15 minutes to 2 days, preferably 1 hour to 4 hours.

In addition to Steps 61 and 62, the method may include, if necessary, a step of protecting functional groups such as a hydroxyl group, a carbonyl group, an amino group, and a carboxyl group, and a step of deprotecting the protected functional groups. Protective groups for these functional groups and protection/deprotection reactions are well known to those skilled in the art, and appropriate protective groups and protection/deprotection reactions can be selected with reference to "Greene's Protective Groups in Organic Synthesis" or the like.

The compound represented by Formula (9a) can be produced by condensing lipoic acid with NHR⁷R⁸. Examples of solvents for the condensation reaction include dichloromethane, chloroform, and tetrahydrofuran, and tetrahydrofuran is preferred. The amount of solvent may be 1 mL to 200 mL, preferably 3 mL to 35 mL, per 1 g of the compound represented by Formula (9a). Examples of condensing agents used include 1-(3-dimethylaminopropyl)-3-ethylcarbodiimide (EDC) and a salt thereof, N,N'-dicyclohexylcarbodiimide (DCC), and diisopropylcarbodiimide (DIC) (N-hydroxysuccinimide (NHS) and 1-hydroxybenzotriazole (HOBt) may be used as additives). The amount of condensing agent used may be 0.8 equivalents to 2.0 equivalents, preferably 1.0 equivalent to 1.5 equivalents, per 1 equivalent of the compound represented by Formula (9a). The reaction temperature may be -10°C to 40°C, preferably 15°C to 25°C. The reaction time may be 1 hour to 3 days, preferably 1 hour to 24 hours.

The compound represented by Formula (9) can also be produced by condensing lipoic acid trisulfide with NHR⁷R⁸. The conditions for condensation are the same as described above.

The compound represented by Formula (7) can be produced by a step (Step 61) of oxidizing a compound represented by Formula (7a) with an oxidizing agent to obtain a sulfoxide compound, and a step (Step 62) of reacting the obtained sulfoxide compound with a sulfur source. The reaction conditions are the same as described above. 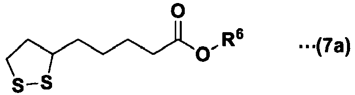 wherein R⁶ is a hydrogen atom; a C₁₋₆ alkyl group optionally having at least one substituent selected from the group consisting of a carboxy group, -OR⁹, -NR¹⁰R¹¹, and -N⁺R¹²R¹³R¹⁴; or -(CH₂CH₂O)ₙR¹⁵, wherein R⁹, R¹⁰, R¹¹, R¹², R¹³, R¹⁴, and R¹⁵ are each independently a hydrogen atom or a C₁₋₃ alkyl group, and n is an integer of 2 to 5.

The compound represented by Formula (7a) can be produced by condensing lipoic acid with R⁶OH. The conditions are the same as described above.

The compound represented by Formula (7) can also be produced by condensing lipoic acid trisulfide with R⁶OH. The conditions for condensation are the same as described above.

In the present invention, examples of pharmaceutically acceptable salts include salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; salts with amino acids such as arginine; and the like. When compound (1) or compound (6) is obtained as a free form, it can be converted to a salt by a conventional method. When compound (1) or compound (6) is obtained as a salt, it can also be converted to a free form by a conventional method.

The trisulfide compound according to the present invention may have crystal polymorphs, but is not limited to any particular crystal form, and may be a single crystal form or a mixture of crystal forms. The trisulfide compound according to the present invention also includes amorphous forms. The trisulfide compound according to the present invention includes anhydrates and solvates (especially hydrates).

The trisulfide compound may be a cyclodextrin clathrate (CD clathrate) in which at least one selected from the group consisting of compound (1) and a pharmaceutically acceptable salt thereof, and compound (6) and a pharmaceutically acceptable salt thereof is included in a cyclodextrin.

The cyclodextrin may be α-cyclodextrin, β-cyclodextrin, γ-cyclodextrin, or a derivative thereof. The term "derivative" means that a hydrogen atom of at least one hydroxyl group of each cyclodextrin is substituted with an alkyl group optionally having a substituent or a saccharide. Examples of cyclodextrin derivatives include methyl-α-cyclodextrin, methyl-β-cyclodextrin, methyl-γ-cyclodextrin, dimethyl-α-cyclodextrin, dimethyl-β-cyclodextrin, dimethyl-γ-cyclodextrin, hydroxyethyl-α-cyclodextrin, hydroxyethyl-β-cyclodextrin, hydroxyethyl-γ-cyclodextrin, 2-hydroxypropyl-α-cyclodextrin, 2-hydroxypropyl-β-cyclodextrin, 2-hydroxypropyl-γ-cyclodextrin, glucosyl-α-cyclodextrin, glucosyl-β-cyclodextrin, glucosyl-γ-cyclodextrin, maltosyl-α-cyclodextrin, maltosyl-β-cyclodextrin, maltosyl-γ-cyclodextrin, sulfobutyl ether-β-cyclodextrin and the like.

The cyclodextrin clathrate can be produced by a step (Step a) of dissolving cyclodextrin in a solvent, a step (Step b) of adding at least one selected from the group consisting of compound (1) and a pharmaceutically acceptable salt thereof, and compound (6) and a pharmaceutically acceptable salt thereof to the obtained solution and stirring, and a step (Step c) of filtering the stirred solution, washing with the same solvent as used in Step a, freezing the filtrate, and freeze-drying. The filtration and washing operation in Step c may be omitted.

The solvent used in Step a is preferably water.

The amount of solvent used in Step a may be 1 to 350 mL, preferably 1 to 80 mL, per 1 g of cyclodextrin.

In Step b, the mass ratio of cyclodextrin to at least one selected from the group consisting of compound (1) and a pharmaceutically acceptable salt thereof, and compound (6) and a pharmaceutically acceptable salt thereof may be 2 to 20, preferably 5 to 16.5.

The stirring temperature in Step b may be 20 to 50°C, and may be room temperature.

The stirring time in Step b may be 0.25 to 40 hours, preferably 2 to 35 hours.

In Step b, after adding at least one selected from the group consisting of compound (1) and a pharmaceutically acceptable salt thereof, and compound (6) and a pharmaceutically acceptable salt thereof, the same solvent as used in Step a may be added before stirring. In this case, the amount of solvent may be 0 to 30 mL, preferably 0 to 20 mL, per 1 g of cyclodextrin.

The amount of solvent used in Step c may be 0 to 150 mL, preferably 0 to 20 mL, per 1 g of cyclodextrin.

The freezing temperature in Step c may be -30 to -20°C, preferably -20°C.

The freezing time in Step c may be 10 to 50 hours.

The freeze-drying in Step c may be carried out at an absolute pressure of 20 to 100 Pa, with an external temperature of 10 to 40°C, preferably an external temperature of 20°C.

The freeze-drying period in Step c may be 1 to 5 days.

The composition for prevention or improvement and the method for prevention or improvement of the present invention may be administered or applied to humans.

Hepatic dysfunction in the present invention refers to a state in which an abnormality is observed in the function of the liver. Specifically, examples include a state in which hepatocytes are damaged and leaked enzymes are leaking into the blood, a state in which a large amount of triglycerides is accumulated in the liver tissue compared to a normal liver, a state in which liver parenchymal cells are degenerated or necrotic, and the like.

The hepatic dysfunction in the present invention may be a condition in which an abnormality is observed in hepatic function without being accompanied by a hepatic disease, or a condition in which an abnormality is observed in hepatic function accompanied by a hepatic disease. The hepatic dysfunction in the present invention may be due to reactive oxygen species-induced damage. Reactive oxygen species-induced damage means damage caused by reactive oxygen species. Examples of hepatic diseases include hepatitis B, hepatitis C, alcoholic liver disease, non-alcoholic fatty liver disease, drug-induced liver disease, chronic hepatitis, and the like.

The composition of the present invention can also prevent or improve symptoms related to hepatic dysfunction. That is, the present invention can also be referred to as a composition for preventing or improving symptoms related to hepatic dysfunction. Examples of symptoms related to hepatic dysfunction include subjective symptoms that occur due to a decline in liver function, such as fatigue, fatigue sensation, malaise, nausea, loss of appetite, gastrointestinal symptoms, jaundice, rough skin, edema, and fever, as well as liver cirrhosis, liver cancer, and their complications.

The composition for prevention or improvement of the present invention can be provided as a food composition for preventing or improving hepatic dysfunction and symptoms related thereto. Furthermore, the composition for prevention or improvement of the present invention can also be provided as a pharmaceutical composition for preventing or improving hepatic dysfunction and symptoms related thereto.

The food composition includes general foods, as well as foods with function claims, foods for specified health uses, and the like. The form of the food composition can be, for example, a form suitable for consumption, such as solid, liquid, granular, powder, capsule, cream, or paste.

When providing the composition for preventing or improving hepatic dysfunction of the present invention as a pharmaceutical composition, it can be mixed with pharmaceutically acceptable additives and formulated into a preparation form suitable for administration. The composition for preventing or improving hepatic dysfunction of the present invention, which is a pharmaceutical composition, can be orally administered, for example, as tablets, capsules, powders, granules, solutions, or syrups, and can also be parenterally administered as injections, infusions, or suppositories. Oral administration is preferable because it allows for convenient administration. These dosage forms can be formulated by known formulation techniques. In the case of a solid preparation, pharmaceutically acceptable excipients, for example, starch, lactose, purified sucrose, glucose, crystalline cellulose, carboxy cellulose, carboxymethyl cellulose, carboxyethyl cellulose, calcium phosphate, magnesium stearate, gum arabic, and the like can be blended during formulation, and if necessary, a lubricant, a binder, a disintegrant, a coating agent, a coloring agent, and the like can be blended. In the case of a liquid preparation, a stabilizer, a solubilizer, a suspending agent, an emulsifier, a buffer, a preservative, and the like can be blended.

The dosage of the composition for preventing or improving hepatic dysfunction of the present invention varies depending on the symptoms, age, administration method, dosage form, and the like, but in a normal case, it can be administered to an adult as the above-mentioned compound at 0.5 to 2000 mg, preferably 10 to 1000 mg, and most preferably 50 to 800 mg per day, and it is preferable to administer 1 to 800 mg of the compound once or in several divided doses per day, for consecutive days. When the dosage of LASSS and LASSS-CE is within these ranges, it is considered that the preventive or improving effect on hepatic dysfunction and symptoms related thereto is further enhanced.

### Examples

A human liver cancer-derived cell line (HepG2: HB-8065^{™}, Lot No. 70029112) was obtained from the American Type Culture Collection, and Dulbecco's Modified Eagle Medium (Thermo Fisher Scientific K.K.) containing 10% FBS was used as the basal medium. The cells were cultured in a 37°C, 5% CO₂ incubator, and cells passaged for 17 to 22 generations were used for the experiments.

A cell suspension adjusted to an initial cell concentration of 2 x 10⁴ cells/mL was seeded at 0.15 mL into a 96-well plate (Thermo Fisher Scientific K.K.).

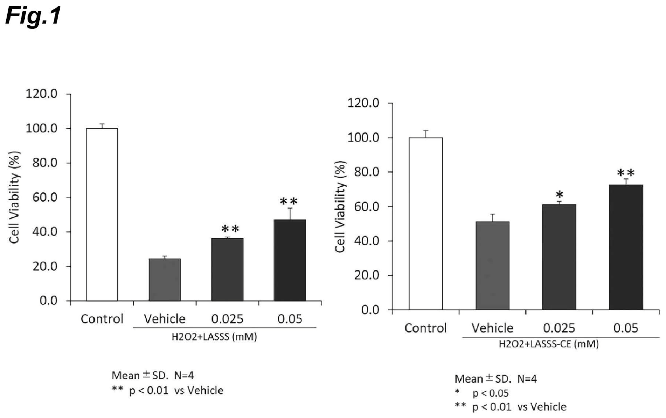

After 20 hours of culture from seeding, alpha-lipoic acid trisulfide (LASSS) or alpha-lipoic acid trisulfide choline ester (LASSS-CE) was added to the medium in groups with a final concentration of 0.025 mM or 0.05 mM. Separately, a group to which only the solvent for LASSS or LASSS-CE (phosphate-buffered saline (PBS) containing 4% DMSO (FUJIFILM Wako Pure Chemical Corporation)) was added was designated as the solvent-added group. After 4 hours of culture following the sample addition, the medium of the above sample-added groups and the solvent-added group was replaced with a medium (not containing LASSS or LASSS-CE) to which hydrogen peroxide solution (FUJIFILM Wako Pure Chemical Corporation) had been added to a final concentration of 0.1 mM, thereby inducing hepatocyte damage. A group in which the same amount of PBS was added instead of hydrogen peroxide solution to the solvent-added group was designated as the control group. After culturing for 2 hours following the addition of hydrogen peroxide solution or PBS, cell viability was measured by the MTT assay to evaluate the extent of hepatocyte injury. The results are shown in Fig. 1 and Table 2. In the table, SD represents standard deviation.

**[Table 2]**

| | LASSS | | LASSS-CE | |
|---|---|---|---|---|
| | Cell viability (%) | SD | Cell viability (%) | SD |
| Control group | 100.0 | 2.7 | 100.0 | 4.3 |
| Hydrogen peroxide-added group | 24.3 | 1.7 | 51.0 | 4.4 |
| Hydrogen peroxide + Sample-added group (0.025 mM) | 36.3 | 0.9 | 61.2 | 1.7 |
| Hydrogen peroxide + Sample-added group (0.05 mM) | 47.1 | 6.6 | 72.4 | 3.6 |

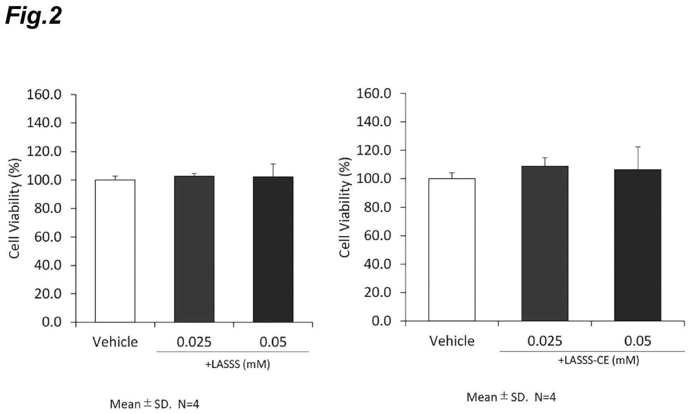

Furthermore, to evaluate the effect of LASSS and LASSS-CE on cell proliferation itself, a similar experiment was conducted by replacing the addition of hydrogen peroxide solution with the addition of the same amount of PBS, and the cell viability was measured by the MTT assay method. The results are shown in Fig. 2 and Table 3.

**[Table 3]**

| | LASSS | | LASSS-CE | |
|---|---|---|---|---|
| | Cell viability (%) | SD | Cell viability (%) | SD |
| Control group | 100.0 | 2.7 | 100 | 4.3 |
| Sample-added group (0.025 mM) | 102.5 | 2.0 | 108.7 | 6.0 |
| Sample-added group (0.05 mM) | 102.2 | 9.0 | 106.3 | 16.0 |

As shown in Fig. 1 and Table 2, as a result of inducing reactive oxygen species-induced damage by the addition of hydrogen peroxide solution, the cell viability of HepG2 cells decreased. In the groups to which LASSS and LASSS-CE were added, an improvement in cell viability was confirmed compared to the hydrogen peroxide-added group. Therefore, it was suggested that LASSS and LASSS-CE have an excellent effect of suppressing or improving damage to the liver due to oxidative stress.

As shown in Fig. 2 and Table 3, under experimental conditions without the influence of hydrogen peroxide solution which causes oxidative stress damage, LASSS and LASSS-CE did not affect the proliferation of HepG2 cells.

## Claims

1. A composition for preventing or improving hepatic dysfunction, comprising a trisulfide compound,
wherein the trisulfide compound is at least one selected from the group consisting of:
a compound represented by Formula (1): 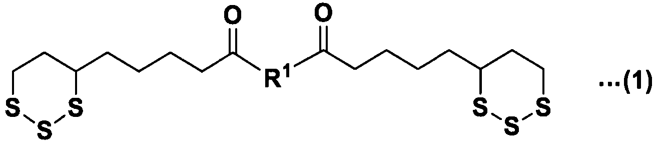
wherein R¹ is a group represented by Formula (2): 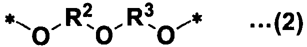
wherein R² and R³ are each independently a C₁₋₄ alkylene group, and * is a bond;
a group represented by Formula (3): 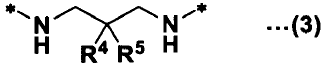
wherein R⁴ and R⁵ are each independently a hydrogen atom or a C₁₋₄ alkyl group optionally having a substituent, and R⁴ and R⁵ may form a ring together with the atom bonded thereto, and * is a bond;
a group represented by Formula (4): 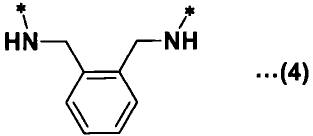
wherein * is a bond, or
a group represented by Formula (5): 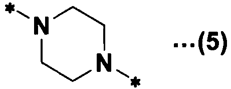
wherein * is a bond,
a pharmaceutically acceptable salt thereof, and a cyclodextrin clathrate thereof; and
a compound represented by Formula (6): 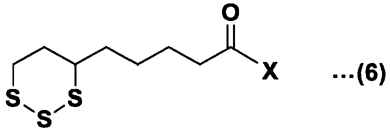
wherein X is -OR⁶ or -NR⁷R⁸,
wherein R⁶, R⁷, and R⁸ are each independently a hydrogen atom; a C₁₋₆ alkyl group optionally having at least one substituent selected from the group consisting of a carboxy group, -OR⁹, -NR¹⁰R¹¹, and - N⁺R¹²R¹³R¹⁴; or -(CH₂CH₂O)ₙR¹⁵,
wherein R⁹, R¹⁰, R¹¹, R¹², R¹³, R¹⁴, and R¹⁵ are each independently a hydrogen atom or a C₁₋₃ alkyl group, and n is an integer of 2 to 5,
a pharmaceutically acceptable salt thereof, and a cyclodextrin clathrate thereof.

2. The composition according to claim 1, wherein the hepatic dysfunction is a condition in which an abnormality is observed in hepatic function without being accompanied by a hepatic disease, or a condition in which an abnormality is observed in hepatic function accompanied by a hepatic disease.

3. The composition according to claim 2, wherein the hepatic disease is at least one selected from the group consisting of hepatitis B, hepatitis C, alcoholic liver disease, non-alcoholic fatty liver disease, and chronic hepatitis.

4. The composition according to claim 1, wherein the hepatic dysfunction is due to reactive oxygen species-induced damage.

5. The composition according to any one of claims 1 to 4, which is a food composition.

6. The composition according to any one of claims 1 to 4, which is a pharmaceutical composition.

7. The composition according to any one of claims 1 to 4, wherein the trisulfide compound is alpha-lipoic acid trisulfide or a pharmaceutically acceptable salt thereof, or alpha-lipoic acid trisulfide choline ester or a pharmaceutically acceptable salt thereof.
